# EUROPEAN PATENT APPLICATION

(11) **EP 4 748 880 A1**
(43) Date of publication of application: **27.05.2026**
(21) Application number: 24842048.1
(22) Date of filing: 21.05.2024
(51) Int. Cl.: C08L 7/00, C08L 9/00, C08K 5/18, C07C 211/51, C07C 211/55

(54) **FATIGUE- AND AGING-RESISTANT RUBBER COMPOSITION AND USE THEREOF**

(30) Priority: 19.07.2023 CN 202310890170
(71) Applicant: Sennics Co., Ltd., Shanghai, 200120 (CN)
(72) Inventor: GAO, Yang, Shanghai 200120 (CN); ZHANG, Jin, Shanghai 200120 (CN); GUO, Xiangyun, Shanghai 200120 (CN)
(74) Representative: V.O.
(86) International application number: PCT/CN2024/094385
(87) International publication number: WO 2025/016048

(57) **Abstract**

The present invention provides a fatigue- and aging-resistant rubber composition and a use thereof. The rubber composition comprises the raw materials of 100 parts by mass of a diene elastomer and 1-3 parts by mass of a compound of formula A. The compound of formula A is as described in the description. The compound of formula A can significantly improve the fatigue and aging resistance of the rubber composition and prolong the service life of rubber products.

## Description

### Technical Field

The present invention belongs to the field of rubber materials, and relates to a fatigue aging-resistant rubber composition and its use.

### Background

Fatigue aging of rubber products refers to damage or irreversible changes in structure and performance occurring under the action of periodic stress and deformation. Tire, transmission belt, conveyor belt, rubber spring, and rubber shock absorber are all prone to fatigue aging. Rubber fatigue is caused by periodic mechanical forces, but this phenomenon does not exist in isolation. Throughout the entire fatigue process, thermal oxidative aging and ozone aging occur simultaneously, resulting from the combined effect of multiple factors. The main external factors comprise ambient temperature, oxygen content, ozone concentration, as well as strain amplitude and frequency. Fatigue aging accelerates the damage of rubber products and shortens their service life. The fatigue aging resistance of rubber is usually defined as the number of periodic stress (deformation) cycles a sample endures before damage occurs. Generally, flex-cracking test and dynamic tensile fatigue test are used to evaluate the fatigue resistance of rubber. The former observes the generation of cracks and the growth of crack mouths in the sample, while the latter records the number of fatigue cycles when the sample breaks.

P-phenylenediamine antidegradants, such as 6PPD (N-(1,3-dimethylbutyl)-N'-phenyl-p-phenylenediamine) and IPPD (N-isopropyl-N'-phenyl-p-phenylenediamine), possess good ozone aging resistance and fatigue aging resistance, thus being widely used in the recipes of automotive tire products.

However, when the external environment and conditions for the use of rubber products become extremely harsh or longer service life is pursued, technicians often simply increase the dosage of antidegradants to meet the performance requirements. Nevertheless, all p-phenylenediamine antidegradants suffer from severe migration and discoloration issues. Increasing the dosage of antidegradants will inevitably further exacerbate problems such as the appearance quality of tires and environmental pollution, while also leading to increased costs for enterprises.

### Summary

In view of the problems existing in the prior art, the present invention provides a rubber composition containing a compound of Formula A, a method for improving the fatigue aging resistance of the rubber composition, and the use of the compound of Formula A in improving the fatigue aging resistance of the rubber composition. The compound of Formula A can significantly enhance the fatigue aging resistance of the rubber composition (including flex-cracking aging resistance and dynamic tensile fatigue resistance) and extend the service life of rubber products.

Specifically, one aspect of the present invention provides a rubber composition, wherein the raw materials of the rubber composition comprise 100 parts by mass of a diene elastomer and 1-3 parts by mass, for example, 1.5-2 parts by mass of a compound of Formula A: 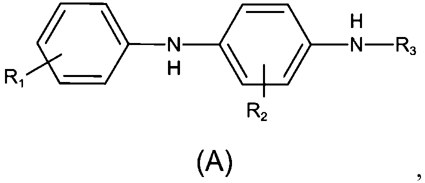 wherein, R₁, R₂, and R₃ in Formula A are each independently selected from C1-C8 alkyl.

In one or more embodiments, the raw materials of the rubber composition comprise two or more compounds of Formula A.

In one or more embodiments, R₁ and R₂ in Formula A are each independently selected from C1-C3 alkyl, preferably each independently selected from methyl and ethyl.

In one or more embodiments, R₃ in Formula A is selected from C3-C8 alkyl, preferably selected from C3-C6 alkyl, for example, selected from C4-C6 alkyl.

In one or more embodiments, the raw materials of the rubber composition comprise one or more compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV:

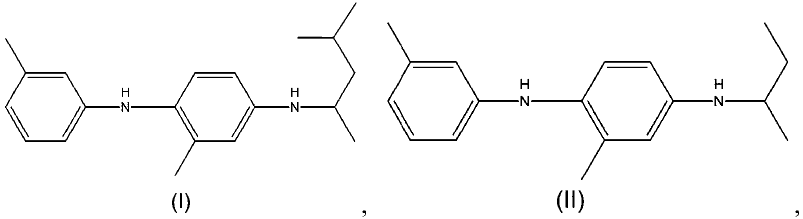

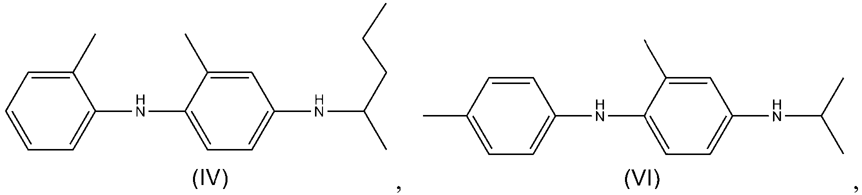

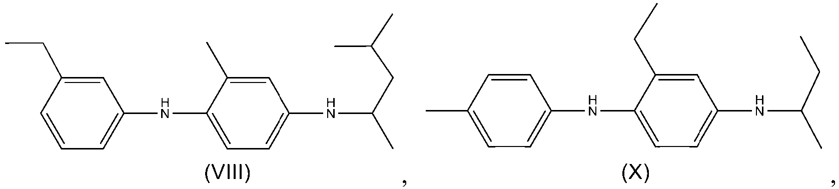

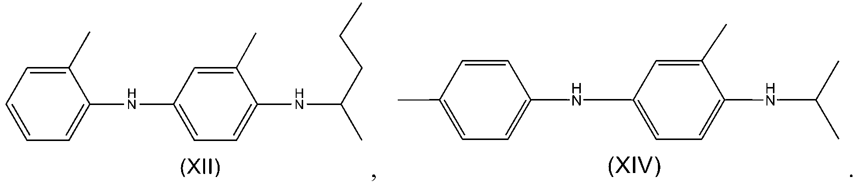

In one or more embodiments, the raw materials of the rubber composition comprise two or more compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV.

In one or more embodiments, the raw materials of the rubber composition comprise two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV.

In one or more embodiments, the raw materials of the rubber composition comprise Compound of Formula I and Compound of Formula II, or Compound of Formula IV and Compound of Formula VI, or Compound of Formula VIII and Compound of Formula X, or Compound of Formula XII and Compound of Formula XIV.

In one or more embodiments, in the raw materials of the rubber composition, the total dosage of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is 1-3 parts by mass, for example, 1.5-2 parts by mass.

In one or more embodiments, in the raw materials of the rubber composition, the mass ratio of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is from 1:2 to 2:1, for example, from 1:1.5 to 1.5:1.

In one or more embodiments, the diene elastomer comprises natural rubber and butadiene rubber in a mass ratio of from 1:2 to 2:1.

In one or more embodiments, the raw materials of the rubber composition further comprise 30-70 parts by mass of a reinforcing filler; the reinforcing filler is preferably carbon black.

In one or more embodiments, the raw materials of the rubber composition further comprise 0.5-3 parts by mass of sulfur.

In one or more embodiments, the raw materials of the rubber composition further comprise 1-10 parts by mass of an activator; preferably, the activator is zinc oxide.

In one or more embodiments, the raw materials of the rubber composition further comprise 2-15 parts by mass of a softener.

In one or more embodiments, the raw materials of the rubber composition further comprise 0.2-2 parts by mass of an accelerator; the accelerator is preferably N-tert-butyl-2-benzothiazolesulfenamide.

Another aspect of the present invention provides a rubber article, which comprises the rubber composition according to any one of the embodiments herein.

In one or more embodiments, the rubber article is a tire.

Another aspect of the present invention provides a method for improving the fatigue aging resistance of a rubber composition, wherein the method comprises: based on 100 parts by mass of a diene elastomer contained in the raw materials of the rubber composition, adding 1-3 parts by mass, for example 1.5-2 parts by mass, of a compound of Formula A to the raw materials of the rubber composition: 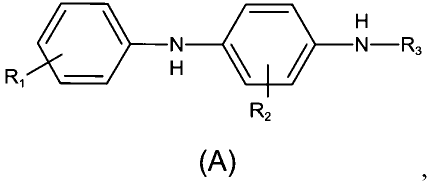 wherein R₁, R₂, and R₃ in Formula A are each independently selected from C1-C8 alkyl.

In one or more embodiments, R₁ and R₂ in Formula A are each independently selected from C1-C3 alkyl, preferably each independently selected from methyl and ethyl.

In one or more embodiments, R₃ in Formula A is selected from C3-C8 alkyl, preferably selected from C3-C6 alkyl, for example, selected from C4-C6 alkyl.

In one or more embodiments, the method comprises: adding two or more compounds of Formula A to the raw materials of the rubber composition.

In one or more embodiments, the method comprises: adding one or more compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV to the raw materials of the rubber composition:

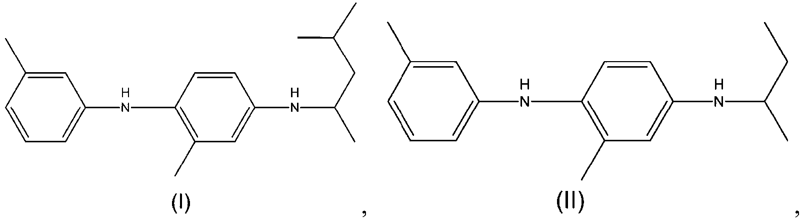

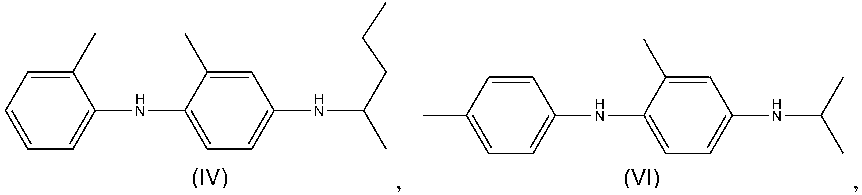

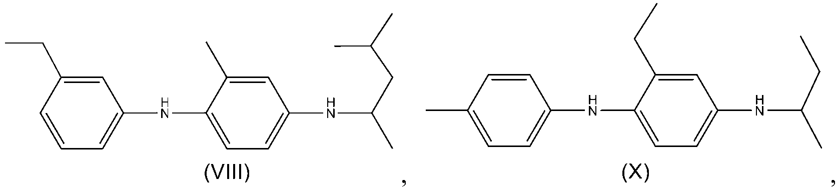

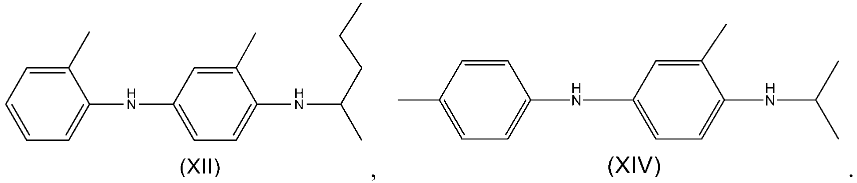

In one or more embodiments, the method comprises: adding two or more compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV to the raw materials of the rubber composition.

In one or more embodiments, the method comprises: adding two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV to the raw materials of the rubber composition.

In one or more embodiments, the method comprises: adding Compound of Formula I and Compound of Formula II, or Compound of Formula IV and Compound of Formula VI, or Compound of Formula VIII and Compound of Formula X, or Compound of Formula XII and Compound of Formula XIV to the raw materials of the rubber composition.

In one or more embodiments, in the raw materials of the rubber composition, the total dosage of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is 1-3 parts by mass, for example, 1.5-2 parts by mass.

In one or more embodiments, in the raw materials of the rubber composition, the mass ratio of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is from 1:2 to 2:1, for example, from 1:1.5 to 1.5:1.

In one or more embodiments, the diene elastomer comprises natural rubber and butadiene rubber in a mass ratio of from 1:2 to 2:1.

In one or more embodiments, the raw materials of the rubber composition further comprise 30-70 parts by mass of a reinforcing filler; the reinforcing filler is preferably carbon black.

In one or more embodiments, the raw materials of the rubber composition further comprise 0.5-3 parts by mass of sulfur.

In one or more embodiments, the raw materials of the rubber composition further comprise 1-10 parts by mass of an activator; preferably, the activator is zinc oxide.

In one or more embodiments, the raw materials of the rubber composition further comprise 2-15 parts by mass of a softener.

In one or more embodiments, the raw materials of the rubber composition further comprise 0.2-2 parts by mass of an accelerator; the accelerator is preferably N-tert-butyl-2-benzothiazolesulfenamide.

### Detailed Description

To enable those skilled in the art to understand the characteristics and effects of the present invention, the following provides general explanations and definitions for the terms and expressions mentioned in the specification and claims. Unless otherwise specified, all technical and scientific terms used herein shall have the ordinary meanings understood by those skilled in the art with respect to the present invention. In case of any conflict, the definitions in this specification shall prevail.

The theories or mechanisms described and disclosed herein, whether correct or incorrect, shall not limit the scope of the present invention in any way, that is, the content of the present invention can be implemented without being restricted by any specific theory or mechanism.

As used herein, the terms "comprise", "include", "contain" and similar expressions encompass the meanings of "consisting essentially of" and "consisting of". For example, when the present invention discloses that "A comprises B and C", "A consists essentially of B and C" and "A consists of B and C" shall be deemed to have been disclosed herein.

In this specification, all features defined in the form of numerical ranges or percentage ranges, such as values, quantities, contents and concentrations, are merely for brevity and convenience. Accordingly, the description of a numerical range or percentage range shall be deemed to have covered and specifically disclosed all possible sub-ranges and individual values (including integers and fractions) within the range.

In this specification, unless otherwise stated, percentages refer to mass percentages, and ratios refer to mass ratios.

When describing embodiments or examples herein, it should be understood that they are not intended to limit the present invention to these embodiments or examples. On the contrary, all alternatives, modifications and equivalents of the methods and materials described in the present invention may be comprised within the scope defined by the claims.

In this specification, for the sake of concise description, not all possible combinations of various technical features in each embodiment or example are described. Therefore, as long as there is no contradiction in the combination of these technical features, the various technical features in the various embodiments or examples can be combined arbitrarily, and all possible combinations should be considered within the scope of this specification.

As used herein, "alkyl" refers to a straight-chain or branched monovalent saturated hydrocarbon group, usually containing 1 to 18 carbon atoms (C1-C18 alkyl), for example, containing 1 to 8 carbon atoms (C1-C8 alkyl). Examples of alkyl comprise, but are not limited to, methyl, ethyl, propyl, isopropyl, 1-methylpropyl, isobutyl, 1-methylbutyl, and 1,3-dimethylbutyl.

In the present invention, the compound of Formula A has the following structure: 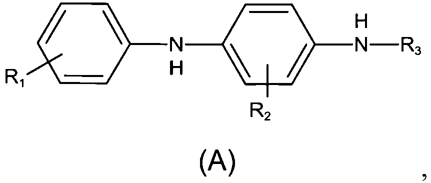 wherein R₁, R₂, and R₃ in Formula A are each independently selected from C1-C8 alkyl.

In some embodiments, R₁ is selected from C1-C4 alkyl, preferably selected from C1-C3 alkyl, for example, selected from methyl and ethyl. R₁ may be located at the ortho-position, meta-position, or para-position of the -NH- group.

In some embodiments, R₂ is selected from C1-C4 alkyl, preferably selected from C1-C3 alkyl, for example, selected from methyl and ethyl. R₂ may be located at the ortho-position or meta-position of the -NH-R₃ group.

In some embodiments, R₃ is selected from C3-C8 alkyl, preferably selected from C3-C6 alkyl, for example, selected from isopropyl, 1-methylpropyl, 1-methylbutyl, and 1,3-dimethylbutyl, more preferably selected from C4-C6 alkyl.

Examples of the compound of Formula A comprise the following compounds of Formula I, Formula II, Formula IV, Formula VI, Formula VIII, Formula X, Formula XII, and Formula XIV:

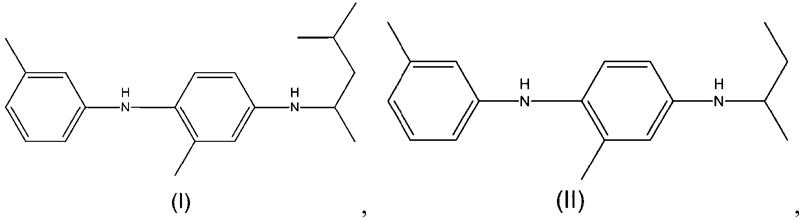

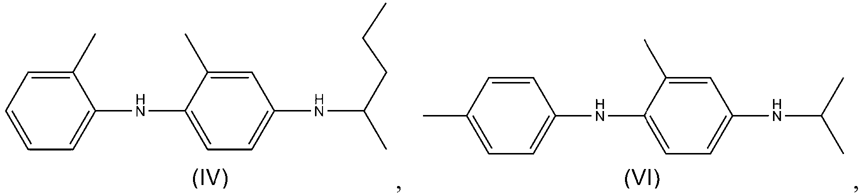

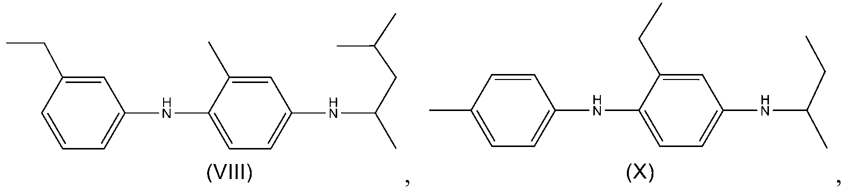

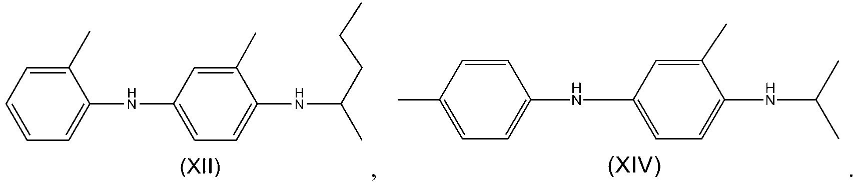

The present invention finds that the use of one or more compounds of Formula A in rubber compositions, especially tire rubber compositions, can significantly improve the fatigue aging resistance of the rubber compositions, and the improvement effect is superior to that of the antidegradant 6PPD. As used herein, the improvement of fatigue aging resistance means that the flex-cracking resistance and/or dynamic tensile fatigue resistance of the rubber composition is enhanced.

The present invention also discovers that compared with the use of a single compound of Formula A, the use of two or more compounds of Formula A exhibits a significant synergistic effect in improving the fatigue aging resistance of the rubber composition. In some embodiments, the present invention uses two compounds of Formula A in the rubber composition, for example, two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV. For instance, Compound of Formula I and Compound of Formula II, or Compound of Formula IV and Compound of Formula VI, or Compound of Formula VIII and Compound of Formula X, or Compound of Formula XII and Compound of Formula XIV, are used in the rubber composition.

In the present invention, the compound of Formula A can be prepared by a method comprising the following steps:
(1) conducting a condensation reaction between a compound of Formula B and a compound of Formula C in the presence of a first catalyst to obtain a condensation containing a compound of Formula D and/or a compound of Formula D', and then subjecting the condensation to a reduction reaction in the presence of H₂ and a second catalyst to obtain a compound of Formula E; 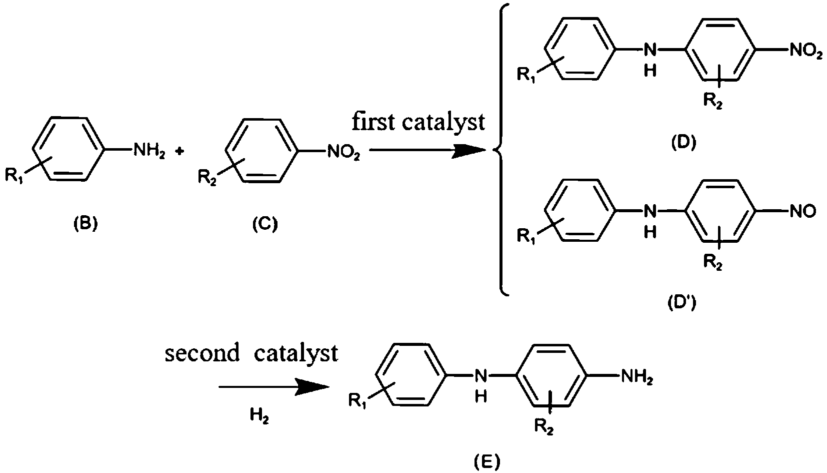
(2) reacting the compound of Formula E with an aldehyde compound or a ketone compound represented by Formula F in the presence of Hz and a third catalyst to carry out a reductive alkylation reaction, thereby obtaining the compound of Formula A.

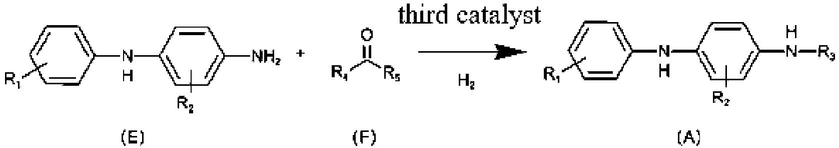

R₁, R₂, and R₃ in Formula A, Formula B, Formula C, Formula D, and Formula E are as described in any embodiment herein. R₄ and R₅ in Formula F are each independently selected from H and C1-C7 alkyl. A person skilled in the art can determine the appropriate R₄ and R₅ in Formula F according to the R₃ contained in the compound of Formula A.

In step (1), the first catalyst used may be one or more selected from the group consisting of alkali metal hydroxides, alkali metal alkoxides, quaternary ammonium bases, and combinations of alkali metal hydroxides and halides of tetraalkylammonium. Alkali metal hydroxides suitable for the present invention comprise sodium hydroxide, potassium hydroxide, lithium hydroxide, and the like. Alkali metal alkoxides suitable for the present invention comprise sodium methoxide, potassium methoxide, sodium ethoxide, potassium ethoxide, sodium tert-butoxide, potassium tert-butoxide, sodium tert-pentoxide, potassium tert-pentoxide, and the like. Quaternary ammonium bases are compounds having the general formula R¹₄NOH, where R represents four identical or different aliphatic hydrocarbon groups or aromatic hydrocarbon groups. In the quaternary ammonium bases suitable for the present invention, R¹ may be one or more selected from methyl, ethyl, propyl, butyl, and the like. Examples of quaternary ammonium bases suitable for the present invention comprise tetramethylammonium hydroxide, tetraethylammonium hydroxide, tetrabutylammonium hydroxide, and the like. The first catalyst may also be a combination of an alkali metal hydroxide and a halide of tetraalkylammonium. Halides of tetraalkylammonium have the general formula R²₄NX, where R² represents four identical or different aliphatic hydrocarbon groups or aromatic hydrocarbon groups, such as methyl, ethyl, propyl, butyl, and the like, and X represents a halogen atom, such as fluorine, chlorine, bromine, or iodine. Examples of combinations of alkali metal hydroxides and halides of tetraalkylammonium comprise sodium hydroxide and tetrabutylammonium bromide, and the like.

The molar ratio of the first catalyst to the compound of Formula B may be from 0.1:1 to 2:1, preferably from 0.9:1 to 1.1:1, for example, 1.05:1, 1.1:1, or 1.5:1. In one or more embodiments, in step (1), the compound of Formula B is first reacted with the first catalyst to form a salt, and then the compound of Formula C is added dropwise to carry out the condensation reaction.

In step (1), the condensate obtained by the condensation reaction of the compound of Formula B and the compound of Formula C in the presence of the first catalyst may be one or both of the compound of Formula D and the compound of Formula D', and may also contain azobenzene compounds.

In step (1), the molar ratio of the compound of Formula B to the compound of Formula C may be from 2:1 to 15:1, preferably from 4:1 to 10:1, and more preferably from 5:1 to 8:1, for example, 6:1 or 7:1.

In step (1), the condensation reaction may be carried out at 40 to 90°C, preferably 65 to 85°C. For example, the reaction temperature may be 60°C, 70°C, 75°C, or 80°C. The condensation reaction needs to be performed under vacuum conditions with a pressure range of -0.09 to -0.1 MPa.

The second catalyst used in step (1) may be a porous metal catalyst or a supported metal catalyst. Porous metal catalysts are also known as spongy metal catalysts. Porous metal catalysts suitable for the present invention comprise Raney nickel (also referred to as skeleton nickel), Raney cobalt, Raney copper, and the like. Supported metal catalysts comprise metals serving as catalytic active centers and supports for loading the metals. The metal in the supported metal catalyst suitable for the present invention may be nickel, cobalt, copper, platinum, palladium, ruthenium, rhodium, etc., and the support may be carbon, alumina, silica gel, molecular sieve, etc. The carbon used as the support may be activated carbon. The molar ratio of the metal in the second catalyst to the condensate may be from 0.0001:1 to 0.2:1.

In step (1), the condensate generated by the condensation reaction undergoes a hydrogenation reduction reaction in the presence of the second catalyst to produce the compound of Formula E. In step (1), the reduction reaction may be carried out at 40 to 120°C, preferably 60 to 90°C. For example, the reaction temperature may be 70°C, 75°C, or 80°C. The hydrogen pressure in the reduction reaction may be 0.5 to 5 MPa, for example, 1 MPa, 1.5 MPa, 2 MPa, or 2.5 MPa.

In step (1), the compound of Formula B itself may be used as a solvent, or solvents such as toluene and xylene may also be used. After the completion of the reaction in step (1), the reaction liquid is filtered, washed with water, and phase-separated. The organic phase is subjected to reduced pressure distillation to remove light components, and the compound of Formula E can be obtained.

In step (2), the third catalyst used may be the aforementioned supported metal catalyst, such as Pt/C. The molar ratio of the metal in the third catalyst to the compound of Formula III may be from 0.0001:1 to 0.2:1.

In step (2), the carbonyl carbon atom in the compound of Formula F is linked to the amino nitrogen atom in the compound of Formula E after the reaction. Therefore, a suitable compound of Formula F can be selected for the reaction according to the R₃ group contained in the compound of Formula A to be prepared. The molar ratio of the compound of Formula F to the compound of Formula E may be from 1:1 to 15:1, for example, 2:1, 3:1, 5:1, 8:1, or 10:1. The reaction temperature in step (2) may range from 40 to 150°C, such as 50°C, 80°C, 100°C, or 120°C. The hydrogen pressure in step (2) may be from 0.5 to 5 MPa, for example, 1 MPa, 1.5 MPa, 2 MPa, or 2.5 MPa.

In step (2), the compound of Formula F, which serves as a reaction raw material, may be used as a solvent. After the completion of the reaction in step (2), the reaction liquid is filtered and subjected to reduced pressure distillation to remove light components, and the compound of Formula A can be obtained.

In the present invention, liquid chromatography (LC) or gas chromatography (GC) may be used to determine whether each step of the reaction has reached the endpoint, thereby determining the appropriate reaction time.

The raw materials of a rubber composition typically comprise a diene elastomer, a reinforcing filler, an antidegradant, and a crosslinking agent. As used herein, the rubber composition comprises unvulcanized rubber and vulcanized rubber. Unvulcanized rubber can be converted into vulcanized rubber through vulcanization (curing).

The raw materials of the rubber composition of the present invention comprise a diene elastomer and one or more compounds of Formula A as antidegradants. Based on 100 parts by mass of the diene elastomer, the total dosage of the compound(s) of Formula A may be 1-3 parts by mass, for example, 1.3 parts by mass, 1.5 parts by mass, 1.8 parts by mass, 2 parts by mass, 2.2 parts by mass, or 2.5 parts by mass. As used herein, unless otherwise specified, the mass parts of other components in the raw materials of the rubber composition are calculated based on 100 parts by mass of the diene elastomer contained in the raw materials of the rubber composition.

As used herein, a diene elastomer refers to an elastomer whose monomers comprise dienes (such as butadiene and isoprene). Diene elastomers suitable for the present invention may be various diene elastomers known in the art, including but not limited to one or more selected from natural rubber (NR), butadiene rubber (BR), isoprene rubber, styrene-butadiene rubber (SBR), chloroprene rubber (CR), nitrile butadiene rubber (NBR), isoprene/butadiene copolymer, isoprene/styrene copolymer, and isoprene/butadiene/styrene copolymer. In some preferred embodiments, the diene elastomer comprises or consists of natural rubber and butadiene rubber. The mass ratio of natural rubber to butadiene rubber is preferably from 1:2 to 2:1, for example, from 1:1.5 to 1.5:1, from 4.5:5.5 to 5.5:4.5, or 1:1.

The raw materials of the rubber composition of the present invention comprise one or more compounds of Formula A, for example, two or more compounds of Formula A. In some embodiments, the rubber composition of the present invention contains no other antidegradants except the compound(s) of Formula A. In one or more embodiments, the raw materials of the rubber composition of the present invention comprise one or more compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV, for example, two or more such compounds. In the raw materials of the rubber composition of the present invention, the dosage of the compound(s) of Formula A may be 1-3 parts by mass, for example, 1.3 parts by mass, 1.5 parts by mass, 1.8 parts by mass, 2 parts by mass, 2.2 parts by mass, 2.5 parts by mass, or within the range of any two of the foregoing values. Controlling the dosage of the compound(s) of Formula A within the aforementioned range is conducive to ensuring the effect of improving fatigue aging resistance at a relatively low dosage.

In preferred embodiments, the number of types of the compound(s) of Formula A in the raw materials of the rubber composition of the present invention is two or more. The combined use of two or more compounds of Formula A in the rubber composition can achieve a significant synergistic effect in improving fatigue aging resistance. In some embodiments, the number of types of the compound(s) of Formula A in the raw materials of the rubber composition of the present invention is two. In some embodiments, the raw materials of the rubber composition of the present invention comprise two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV. In some embodiments, the raw materials of the rubber composition of the present invention comprise Compound of Formula I and Compound of Formula II. In some embodiments, the raw materials of the rubber composition of the present invention comprise Compound of Formula IV and Compound of Formula VI. In some embodiments, the raw materials of the rubber composition of the present invention comprise Compound of Formula VIII and Compound of Formula X. In some embodiments, the raw materials of the rubber composition of the present invention comprise Compound of Formula XII and Compound of Formula XIV. In the present invention, the mass ratio of the two compounds of Formula A is preferably from 1:2 to 2:1, for example, 1:1.5, 1:1.2, 1:1, 1.2:1, 1.5:1, or within the range of any two of the foregoing values. Controlling the mass ratio of the two compounds of Formula A within the aforementioned range is conducive to obtaining the synergistic effect of improving fatigue aging resistance. In a preferred embodiment, the raw materials of the rubber composition of the present invention comprise Compound of Formula I and Compound of Formula II. The total mass of Compound of Formula I and Compound of Formula II may be 1-3 parts by mass, for example, 1.5-2 parts by mass, and the mass ratio of Compound of Formula I to Compound of Formula II may be from 1:2 to 2:1, for example, from 1:1.5 to 1.5:1.

The raw materials of the rubber composition of the present invention may comprise a reinforcing filler. In the raw materials of the rubber composition of the present invention, the dosage of the reinforcing filler may be 30-70 parts by mass, for example, 40 parts by mass, 45 parts by mass, 50 parts by mass, 55 parts by mass, or 60 parts by mass. Reinforcing fillers suitable for the present invention may be conventional reinforcing fillers used in rubber compositions, including but not limited to one or more selected from carbon black, silica, titanium oxide, calcium carbonate, magnesium carbonate, aluminum hydroxide, magnesium hydroxide, clay, and talc. In some preferred embodiments, the reinforcing filler comprises carbon black, or the reinforcing filler is carbon black.

The raw materials of the rubber composition of the present invention contain a crosslinking agent, such as sulfur. In the raw materials of the rubber composition of the present invention, the dosage of sulfur may be 0.5-3 parts by mass, for example, 1 part by mass, 1.5 parts by mass, 2 parts by mass, or 2.5 parts by mass. The sulfur may be sublimed sulfur.

The raw materials of the rubber composition of the present invention may also comprise other components commonly used in rubber compositions, including but not limited to one or more selected from softeners, protective waxes, activators, and accelerators.

Softeners can be used to improve the processability of the rubber composition. Softeners may comprise petroleum-based softeners, such as naphthenic oil, aromatic oil, processing oil, lubricating oil, paraffin, liquid paraffin, petroleum asphalt, and petrolatum, etc., and may also comprise fatty oil-based softeners, such as stearic acid, castor oil, linseed oil, rapeseed oil, coconut oil, waxes (e.g., beeswax, carnauba wax, and lanolin), tall oil, linoleic acid, palmitic acid, and lauric acid, etc. In the raw materials of the rubber composition of the present invention, the dosage of the softener may be 2-15 parts by mass, for example, 3 parts by mass, 5 parts by mass, 7 parts by mass, 8 parts by mass, 9 parts by mass, 10 parts by mass, 11 parts by mass, or 13 parts by mass. In some preferred embodiments, the softener comprises or consists of aromatic oil and stearic acid. The mass ratio of aromatic oil to stearic acid may be from 1:1 to 5:1, for example, 2:1, 3:1, 3.5:1, or 4:1. In some preferred embodiments, in the raw materials of the rubber composition of the present invention, the dosage of aromatic oil is 1-10 parts by mass, for example, 5 parts by mass, 6 parts by mass, 7 parts by mass, 8 parts by mass, or 9 parts by mass; the dosage of stearic acid is 1-5 parts by mass, for example, 1.5 parts by mass, 2 parts by mass, or 3 parts by mass.

Protective wax can migrate from the interior of the rubber to the surface to form a wax film, which serves to isolate the rubber surface from the external environment. Protective wax is optionally added. When the raw materials of the rubber composition of the present invention comprise protective wax, the dosage of the protective wax may be 1-5 parts by mass, for example, 1.5 parts by mass, 2 parts by mass, 3 parts by mass, or 4 parts by mass.

Activators can accelerate the vulcanization rate and improve the thermal conductivity, wear resistance, tear resistance, etc., of the rubber. In the raw materials of the rubber composition of the present invention, the dosage of the activator may be 1-10 parts by mass, for example, 2 parts by mass, 3 parts by mass, 4 parts by mass, 5 parts by mass, 6 parts by mass, 7 parts by mass, 8 parts by mass, or 9 parts by mass. In some preferred embodiments, the activator comprises ZnO, or the activator is ZnO. In some preferred embodiments, in the raw materials of the rubber composition of the present invention, the dosage of ZnO is 3-8 parts by mass, for example, 4 parts by mass, 5 parts by mass, 6 parts by mass, or 7 parts by mass.

Accelerators are generally vulcanization accelerators, which may be one or more selected from sulfonamide vulcanization accelerators, thiazole vulcanization accelerators, thiuram vulcanization accelerators, thiourea vulcanization accelerators, guanidine vulcanization accelerators, dithiocarbamate vulcanization accelerators, aldimine vulcanization accelerators, aldehyde-ammonia vulcanization accelerators, imidazoline vulcanization accelerators, and xanthate acid vulcanization accelerators. In the raw materials of the rubber composition of the present invention, the dosage of the accelerator may be 0.2-2 parts by mass, for example, 0.5 parts by mass, 0.6 parts by mass, 0.8 parts by mass, 1 part by mass, or 1.5 parts by mass. In some preferred embodiments, the accelerator is accelerator NS (N-tert-butyl-2-benzothiazolesulfenamide).

In addition, if necessary, plasticizers may also be used in the rubber composition, such as DMP (dimethyl phthalate), DEP (diethyl phthalate), DBP (dibutyl phthalate), DHP (diheptyl phthalate), DOP (dioctyl phthalate), DINP (diisononyl phthalate), DIDP (diisodecyl phthalate), BBP (butyl benzyl phthalate), DWP (dilauryl phthalate), and DCHP (dicyclohexyl phthalate), etc. The dosage of the plasticizer may be a conventional dosage in the art.

In some preferred embodiments, the raw materials of the rubber composition of the present invention comprise or consist of: 100 parts by mass of a diene elastomer, 0.5-3 parts by mass of sulfur, 1-3 parts by mass of the compound of Formula A, 30-70 parts by mass of carbon black, 1-10 parts by mass of ZnO, 1-5 parts by mass of stearic acid, 1-10 parts by mass of aromatic oil, and 0.2-2 parts by mass of an accelerator. The diene elastomer preferably comprises natural rubber and butadiene rubber in a mass ratio of 1:2 to 2:1, and the accelerator is preferably accelerator NS.

The unvulcanized rubber of the present invention can be prepared by a conventional rubber mixing method, for example, a two-stage mixing method: First-stage thermomechanical mixing (e.g., in an internal mixer): mixing the raw materials of the rubber composition except for the crosslinking agent and the accelerator, and kneading the entire mixture until a maximum temperature between 110°C and 190°C is reached to obtain the first-stage rubber; Second-stage thermomechanical mixing (e.g., in an open mill): after cooling the first-stage rubber, mixing the first-stage rubber with the crosslinking agent and the accelerator until a maximum temperature below 110°C is reached to obtain the second-stage rubber, i.e., the unvulcanized rubber.

Vulcanizing (curing) the unvulcanized rubber can yield vulcanized rubber. The vulcanization temperature is usually 130°C to 200°C, for example, 140-160°C or 145±5°C. The vulcanization time depends on the vulcanization temperature, vulcanization system, and vulcanization kinetics, and is usually 15-60 minutes, for example, 20-40 minutes or 30±5 minutes.

The rubber composition of the present invention is used in rubber products, especially tires. Compared with the sole use of the antidegradant 6PPD, the use of the compound(s) of Formula A, particularly two or more compounds of Formula A (e.g., Compound of Formula I and Compound of Formula II), can significantly improve the fatigue aging resistance of the rubber products. Therefore, the present invention also provides a rubber product containing the rubber composition described herein. The rubber product may be a tire, a rubber overshoe, a sealing strip, an acoustic panel, a crash pad, etc. The rubber product is preferably a tire, such as sidewall rubber, tread rubber, etc.

The present invention also provides the use of the compound of Formula A in improving the fatigue aging resistance of a rubber composition, as well as a method for improving the fatigue aging resistance of a rubber composition. Preferably, the use or method of the present invention comprises: adding 1-3 parts by mass of the compound of Formula A to the raw materials of the rubber composition, based on 100 parts by mass of the diene elastomer contained in the rubber composition. In the use or method of the present invention, the dosage and ratio of the compound(s) of Formula A, the preferred compounds of Formula A, and the raw material composition of the rubber composition are preferably as described in any embodiment herein.

Hereinafter, the present invention will be described by way of specific examples. It should be understood that these examples are merely illustrative and are not intended to limit the scope of the present invention. Unless otherwise specified, the methods, reagents, and materials used in the examples are conventional methods, reagents, and materials in the art. The raw material compounds in the examples are all commercially available.

The sources of raw materials used in the examples are as follows: natural rubber (SCRS) from Xishuangbanna Sinochem Rubber Co., Ltd.; butadiene rubber (BR9000) from Shandong Yuhuang Chemical Co., Ltd.; antidegradant 6PPD from Sennics Co., Ltd.; carbon black N550, zinc oxide, aromatic oil, stearic acid, sublimed sulfur, and accelerator NS are all common raw materials in the rubber industry.

### Preparation Example 1: Synthesis of Compound of Formula I

### (1) Synthesis of Compound of Formula III

132.4 g (1.23 mol) of m-toluidine and 87.6 g (0.24 mol) of a 25% aqueous solution of tetramethylammonium hydroxide (TMAOH) were added to a 500 mL four-necked flask. The mixture was stirred and heated to 40-50°C, and dehydrated by distillation under reduced pressure to form a salt of TMAOH and m-toluidine. During this process, the color of the reaction solution gradually changed from yellow to dark red. The temperature was gradually raised to 72°C. When the distillate volume reached approximately 50% of the feeding amount of the 25% tetramethylammonium hydroxide catalyst, 30 g (0.22 mol) of m-nitrotoluene was added dropwise while performing distillation under reduced pressure (-0.098 MPa) at 72°C. The dropwise addition took about 3 hours. After the completion of dropwise addition, the temperature was maintained for 1 hour. The reaction was monitored by LC until m-nitrotoluene was completely reacted, and a condensate was obtained.

The above condensate was transferred to a 500 mL stainless steel reactor, followed by the addition of 50 g of deionized water and 40 g of skeleton nickel catalyst. The atmosphere was replaced with hydrogen three times, the temperature was raised to 75°C, and the pressure was increased to 1.5 MPa for reaction. The reaction was monitored by LC until the nitro and nitroso compounds were completely reduced. The reaction solution was filtered, washed with water, and phase-separated. The organic phase was subjected to reduced pressure distillation (-0.1 MPa, 160°C) to remove light components, yielding 37.1 g of the compound of Formula III (yield: about 80%), with a GC-detected purity of >99.5%.

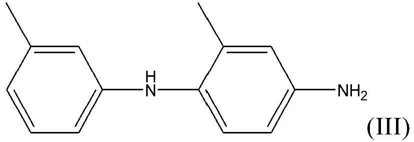

LC-MS(m/z): 212.22(M-H⁺).

¹H NMR (400 MHz, DMSO-*d*₆) δ 6.95 - 6.88 (m, 2H), 6.79 (d, *J* = 8.3 Hz, 1H), 6.48 (d, *J* = 2.6 Hz, 1H), 6.43 - 6.30 (m, 4H), 4.80 (s, 2H), 2.14 (s, 3H), 2.02 (s, 3H).

### (2) Synthesis of Compound of Formula I

37.1 g of the compound of Formula III, 60 g (0.60 mol) of 4-methyl-2-pentanone, and 0.5 g of Pt/C catalyst were charged into a reactor. The atmosphere was replaced with hydrogen three times, the temperature was raised to 100°C, and the pressure was increased to 1.5 MPa for reaction. The reaction was monitored by GC, and when the content of the compound of Formula III was < 0.1%, the reaction was stopped. After cooling down, the catalyst was removed by filtration, and the light components were removed by distillation at -0.1 MPa and 180°C to obtain 49.2 g of the compound of Formula I (yield: about 95%), with a GC-detected purity of > 98.5%. Property: reddish-brown solid.

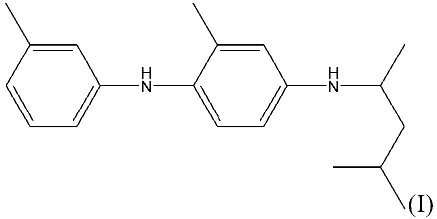

LC-MS(m/z): 296.44(M-H⁺).

### Preparation Example 2: Synthesis of Compound of Formula II

### (1) Synthesis of Compound of Formula III

The synthesis of the compound of Formula III is the same as that in Preparation Example 1.

### (2) Synthesis of Compound of Formula II

30 g (0.14 mol) of the compound of Formula III, 100 g (1.38 mol) of 2-butanone, and 0.6 g of Pt/C catalyst were charged into a reactor. The atmosphere was replaced with hydrogen three times, the temperature was raised to 80°C, and the pressure was increased to 1.5 MPa for reaction. The reaction was monitored by GC, and when the content of the compound of Formula III was < 0.1%, the reaction was stopped. After cooling down, the catalyst was removed by filtration, and the light components were removed by distillation at -0.1 MPa and 150°C to obtain 36.7 g of the compound of Formula II (yield: about 98%), with a GC-detected purity of > 98.5%. Property: reddish-brown solid.

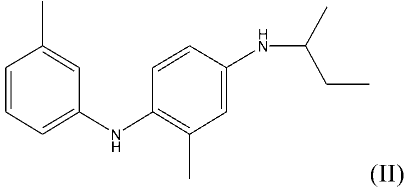

LC-MS(m/z): 268.40 (M-H⁺).

¹H NMR (400 MHz, DMSO-*d*₆) δ 6.96 - 6.87 (m, 2H), 6.83 (d, *J* = 8.4 Hz, 1H), 6.45 (d, *J* = 2.6 Hz, 1H), 6.42 - 6.29 (m, 4H), 4.98 (d, *J* = 8.6 Hz, 1H), 2.14 (s, 3H), 2.04 (s, 3H), 1.81 - 1.66 (m, *J* = 6.7 Hz, 1H), 1.45 (dt, *J* = 13.9, 7.1 Hz, 1H), 1.21 (dt, *J* = 13.5, 6.8 Hz, 1H), 0.89 (dd, *J* = 16.2, 6.6 Hz, 6H).

### Preparation Example 3: Synthesis of Compound of Formula IV

### (1) Synthesis of Compound of Formula V

160.5 g (1.5 mol) of 2-methylaniline and 91 g (0.25 mol) of a 25% aqueous solution of tetramethylammonium hydroxide (TMAOH) were added to a 500 mL four-necked flask. The mixture was stirred and heated to 40-50°C, and dehydrated by distillation under reduced pressure to form a salt of TMAOH and 2-methylaniline. During this process, the color of the reaction solution gradually changed from yellow to dark red. The temperature was gradually raised to 72°C. When the distillate volume reached approximately 50% of the feeding amount of the 25% tetramethylammonium hydroxide catalyst solution, 34.3 g (0.25 mol) of 3-methylnitrobenzene was added dropwise while performing distillation under reduced pressure (-0.098 MPa) at 72°C. The dropwise addition took about 3 hours. After the completion of dropwise addition, the temperature was maintained for 1 hour. The reaction was monitored by LC until 3-methylnitrobenzene was completely reacted, and a condensate was obtained.

The above condensate was transferred to a 500 mL stainless steel reactor, followed by the addition of 50 g of deionized water and 40 g of skeleton nickel catalyst. The atmosphere was replaced with hydrogen three times, the temperature was raised to 75°C, and the pressure was increased to 1.5 MPa for reaction. The reaction was monitored by LC until the nitro and nitroso compounds were completely reduced. Then, the reaction solution was filtered, washed with water, and phase-separated. The organic phase was subjected to reduced pressure distillation (-0.1 MPa, 170°C) to remove light components, yielding 42.8 g of the compound of Formula V (yield: about 80%), with a GC-detected purity of >98%. It is a pale yellow solid at room temperature.

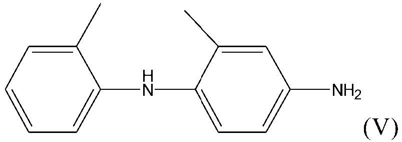

LC-MS(m/z): 212.28 (M-H⁺).

¹H NMR (400 MHz, CDCl₃) δ 7.09 (d, J = 7.3 Hz, 1H), 6.99 (t, J = 7.7 Hz, 1H), 6.91 (d, J = 8.2 Hz, 1H), 6.69 (t, J = 7.3 Hz, 1H), 6.55 (s, 1H), 6.49 (dd, J = 14.3, 5.2 Hz, 2H), 4.91 (s, 1H), 3.48 (br s, 2H), 2.23 (s, 3H), 2.11 (s, 3H).

### (2) Synthesis of Compound of Formula IV

42.5 g (0.2 mol) of the compound of Formula V, 129 g (1.5 mol) of 2-pentanone, and 0.5 g of Pt/C catalyst were charged into a reactor. The atmosphere was replaced with hydrogen three times, the temperature was raised to 90°C, and the pressure was increased to 1.5 MPa for reaction. The reaction was monitored by GC, and when the content of the compound of Formula V was < 0.1%, the reaction was stopped. After cooling down, the catalyst was removed by filtration, and the light components were removed by distillation at -0.1 MPa and 180°C to obtain 53.6 g of the compound of Formula IV (yield: about 95%), with a GC-detected purity of >98.7%. Property: reddish-brown solid.

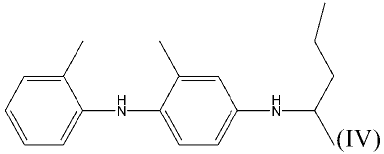

LC-MS(m/z): 282.40 (M-H⁺).

### Preparation Example 4: Synthesis of Compound of Formula VI

### (1) Synthesis of Compound of Formula VII

160.5 g (1.5 mol) of 4-methylaniline and 100.1 g (0.275 mol) of a 25% aqueous solution of tetramethylammonium hydroxide (TMAOH) were added to a 500 mL four-necked flask. The mixture was stirred and heated to 40-50°C, and dehydrated by distillation under reduced pressure to form a salt of TMAOH and 4-methylaniline. During this process, the color of the reaction solution gradually changed from yellow to dark red. The temperature was gradually raised to 72°C. When the distillate volume reached approximately 50% of the feeding amount of the 25% tetramethylammonium hydroxide catalyst solution, 34.3 g (0.25 mol) of 3-methylnitrobenzene was added dropwise while performing distillation under reduced pressure (-0.098 MPa) at 72°C. The dropwise addition took about 3 hours. After the completion of dropwise addition, the temperature was maintained for 1 hour. The reaction was monitored by LC until 3-methylnitrobenzene was completely reacted, and a condensate was obtained.

The above condensate was transferred to a 500 mL stainless steel reactor, followed by the addition of 50 g of deionized water and 40 g of skeleton nickel catalyst. The atmosphere was replaced with hydrogen three times, the temperature was raised to 75°C, and the pressure was increased to 1.5 MPa for reaction. The reaction was monitored by LC until the nitro and nitroso compounds were completely reduced. Then, the reaction solution was filtered, washed with water, and phase-separated. The organic phase was subjected to reduced pressure distillation (-0.1 MPa, 170°C) to remove light components, yielding 46.1 g of the compound of Formula VII (yield: about 86%), with a GC-detected purity of >98%. It is a pale yellow solid at room temperature.

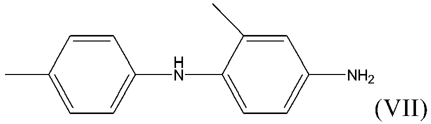

¹H NMR (400 MHz, CDCl₃) δ 7.01 (t, J = 6.6 Hz, 3H), 6.67 - 6.63 (m, 2H), 6.62 (d, J = 2.5 Hz, 1H), 6.55 (dd, J = 8.3, 2.6 Hz, 1H), 5.02 (s, 1H), 3.38 (s, 2H), 2.29 (s, 3H), 2.19 (s, 3H).

¹³C NMR (101 MHz, CDCl₃) δ 144.36, 142.87, 133.94, 132.41, 129.72, 127.73, 125.32, 117.69, 114.90, 113.67, 20.47, 17.99.

### (2) Synthesis of Compound of Formula VI

42.5 g (0.2 mol) of the compound of Formula VII, 162.4 g (2.8 mol) of acetone, and 0.5 g of Pt/C catalyst were charged into a reactor. The atmosphere was replaced with hydrogen three times, the temperature was raised to 70°C, and the pressure was increased to 1.5 MPa for reaction. The reaction was monitored by GC, and when the content of the compound of Formula VII was < 0.1%, the reaction was stopped. After cooling down, the catalyst was removed by filtration, and the light components were removed by distillation at -0.1 MPa and 180°C to obtain 48.8 g of the compound of Formula VI (yield: about 96%), with a GC-detected purity of >98.9%. Property: reddish-brown solid.

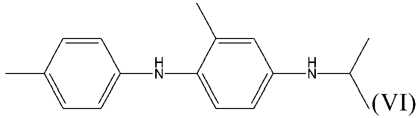

LC-MS(m/z): 254.30 (M-H⁺).

### Preparation Example 5: Synthesis of Compound of Formula VIII

### (1) Synthesis of Compound of Formula IX

149 g (1.23 mol) of m-ethylaniline and 87.4 g (0.24 mol) of a 25% aqueous solution of tetramethylammonium hydroxide (TMAOH) were added to a 500 mL four-necked flask. The mixture was stirred and heated to 40-50°C, and dehydrated by distillation under reduced pressure to form a salt of TMAOH and p-toluidine. During this process, the color of the reaction solution gradually changed from yellow to dark red. The temperature was gradually raised to 72°C. When the distillate volume reached approximately 50% of the feeding amount of the 25% tetramethylammonium hydroxide catalyst, 30.2 g (0.22 mol) of m-nitrotoluene was added dropwise while performing distillation under reduced pressure (-0.098 MPa) at 72°C. The dropwise addition took about 3 hours. After the completion of dropwise addition, the temperature was maintained for 1 hour. The reaction was monitored by LC until m-nitrotoluene was completely reacted, and a condensate was obtained.

The above condensate was transferred to a 500 mL stainless steel reactor, followed by the addition of 50 g of deionized water and 40 g of skeleton nickel catalyst. The atmosphere was replaced with hydrogen three times, the temperature was raised to 75°C, and the pressure was increased to 1.5 MPa for reaction. The reaction was monitored by LC until the nitro and nitroso compounds were completely reduced. The reaction solution was filtered, washed with water, and phase-separated. The organic phase was subjected to reduced pressure distillation (-0.1 MPa, 160°C) to remove light components, yielding 40.3 g of the compound of Formula IX (yield: about 81%), with a GC-detected purity of >97.2%.

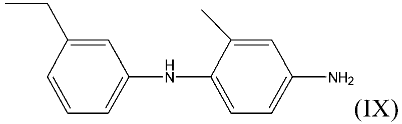

LC-MS(m/z): 226.20 (M-H⁺).

### (2) Synthesis of Compound of Formula VIII

38.5 g (0.17 mol) of the compound of Formula IX, 100 g (1.0 mol) of 4-methyl-2-pentanone, and 0.5 g of Pt/C catalyst were charged into a reactor. The atmosphere was replaced with hydrogen three times, the temperature was raised to 100°C, and the pressure was increased to 1.5 MPa for reaction. The reaction was monitored by GC, and when the content of the compound of Formula IX was < 0.1%, the reaction was stopped. After cooling down, the catalyst was removed by filtration, and the light components were removed by distillation at -0.1 MPa and 180°C to obtain 51 g of the compound of Formula VIII (yield: about 96.5%), with a GC-detected purity of >99.1%. Property: red liquid.

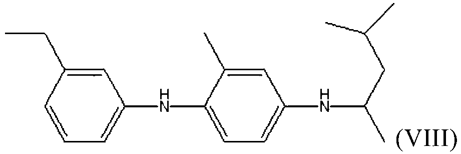

LC-MS(m/z): 310.46 (M-H⁺).

### Preparation Example 6: Synthesis of Compound of Formula X

### (1) Synthesis of Compound of Formula XI

131.8 g (1.23 mol) of p-toluidine and 87.4 g (0.24 mol) of a 25% aqueous solution of tetramethylammonium hydroxide (TMAOH) were added to a 500 mL four-necked flask. The mixture was stirred and heated to 40-50°C, and dehydrated by distillation under reduced pressure to form a salt of TMAOH and p-toluidine. During this process, the color of the reaction solution gradually changed from yellow to dark red. The temperature was gradually raised to 72°C. When the distillate volume reached approximately 50% of the feeding amount of the 25% tetramethylammonium hydroxide catalyst, 33.2 g (0.22 mol) of m-nitroethylbenzene was added dropwise while performing distillation under reduced pressure (-0.098 MPa) at 72°C. The dropwise addition took about 3 hours. After the completion of dropwise addition, the temperature was maintained for 1 hour. The reaction was monitored by LC until m-nitrotoluene was completely reacted, and a condensate was obtained.

The above condensate was transferred to a 500 mL stainless steel reactor, followed by the addition of 50 g of deionized water and 40 g of skeleton nickel catalyst. The atmosphere was replaced with hydrogen three times, the temperature was raised to 75°C, and the pressure was increased to 1.5 MPa for reaction. The reaction was monitored by LC until the nitro and nitroso compounds were completely reduced. The reaction solution was filtered, washed with water, and phase-separated. The organic phase was subjected to reduced pressure distillation (-0.1 MPa, 160°C) to remove light components, yielding 42 g of the compound of Formula XI (yield: about 84.5%), with a GC-detected purity of >97%.

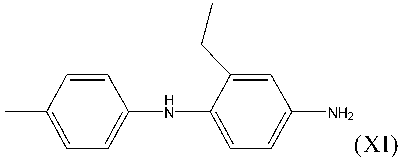

LC-MS(m/z): 226.20 (M-H⁺).

### (2) Synthesis of Compound of Formula X

40.7 g (0.18 mol) of the compound of Formula XI, 108 g (1.50 mol) of 2-butanone, and 0.5 g of Pt/C catalyst were charged into a reactor. The atmosphere was replaced with hydrogen three times, the temperature was raised to 80°C, and the pressure was increased to 1.5 MPa for reaction. The reaction was monitored by GC, and when the content of the compound of Formula XI was < 0.1%, the reaction was stopped. After cooling down, the catalyst was removed by filtration, and the light components were removed by distillation at -0.1 MPa and 180°C to obtain 49.8 g of the compound of Formula X (yield: about 98%), with a GC-detected purity of >98.2%. Property: red solid.

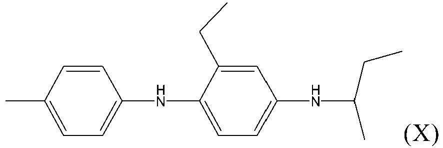

LC-MS(m/z): 282.21(M-H⁺).

### Preparation Example 7: Synthesis of Compound of Formula XII

### (1) Synthesis of Compound of Formula XIII

347.9 g (3.25 mol) of o-toluidine and 200 g (0.55 mol) of a 25% aqueous solution of tetramethylammonium hydroxide (TMAOH) were added to a 1000 mL four-necked flask. The mixture was stirred and heated to 60°C, and dehydrated by distillation under reduced pressure to form a salt of TMAOH and o-toluidine. During this process, the color of the reaction solution gradually changed from yellow to reddish-brown. The temperature was gradually raised to 80°C. When the distillate volume reached approximately half of the feeding amount of the 25% tetramethylammonium hydroxide catalyst, 68.5 g (0.50 mol) of o-nitrotoluene was added dropwise while performing distillation under reduced pressure (-0.097 MPa) at 80°C. The dropwise addition took about 3 hours. After the completion of dropwise addition, the temperature was maintained for 1 hour. The reaction was monitored by LC until o-nitrotoluene was completely reacted, and a condensate was obtained.

The above condensate was transferred to a 500 mL stainless steel reactor, followed by the addition of 65 g of deionized water and 38 g of skeleton nickel catalyst. The atmosphere was replaced with hydrogen three times, the temperature was raised to 78°C, and the pressure was increased to 2.0 MPa for reaction. The reaction was monitored by LC until the nitro and nitroso compounds were completely reduced. The reaction solution was filtered, washed with water, and phase-separated. The organic phase was subjected to reduced pressure distillation (-0.1 MPa, 180°C) to remove light components, yielding 31.8 g of the compound of Formula XIII (yield: about 30%), with a GC-detected purity of >92.5%.

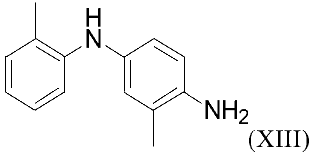

LC-MS(m/z): 212.20(M-H+)

### (2) Synthesis of Compound of Formula XII

30 g (0.14 mol) of the compound of Formula XIII, 120.6 g (1.4 mol) of 2-pentanone, and 0.8 g of Pt/C catalyst were charged into a reactor. The temperature was raised to 90°C, the atmosphere was replaced with hydrogen, and the pressure was increased to 1.5 MPa for reaction. The reaction was monitored by GC, and when the content of the compound of Formula XIII was < 0.1%, the temperature was cooled down and the reaction was stopped. After filtration, the light components were removed by reduced pressure distillation (-0.1 MPa, 160°C) to obtain 38 g of the compound of Formula XII (yield: about 96%), with a GC-detected purity of >96.2%. Property: black solid.

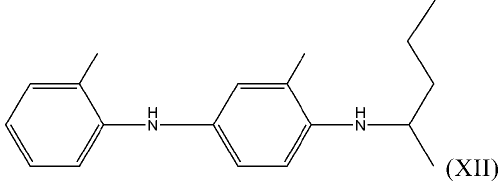

LC-MS(m/z): 282.43(M-H⁺).

### Preparation Example 8: Synthesis of Compound of Formula XIV

### (1) Synthesis of Compound of Formula XV

347.9 g (3.25 mol) of p-toluidine and 200 g (0.55 mol) of a 25% aqueous solution of tetramethylammonium hydroxide (TMAOH) were added to a 1000 mL four-necked flask. The mixture was stirred and heated to 60°C, and dehydrated by distillation under reduced pressure to form a salt of TMAOH and o-toluidine. During this process, the color of the reaction solution gradually changed from yellow to reddish-brown. The temperature was gradually raised to 80°C. When the distillate volume reached approximately half of the feeding amount of the 25% tetramethylammonium hydroxide catalyst, 68.5 g (0.50 mol) of o-nitrotoluene was added dropwise while performing distillation under reduced pressure (-0.097 MPa) at 80°C. The dropwise addition took about 3 hours. After the completion of dropwise addition, the temperature was maintained for 1 hour. The reaction was monitored by LC until o-nitrotoluene was completely reacted, and a condensate was obtained.

The above condensate was transferred to a 500 mL stainless steel reactor, followed by the addition of 65 g of deionized water and 38 g of skeleton nickel catalyst. The atmosphere was replaced with hydrogen three times, the temperature was raised to 78°C, and the pressure was increased to 2.0 MPa for reaction. The reaction was monitored by LC until the nitro and nitroso compounds were completely reduced. The reaction solution was filtered, washed with water, and phase-separated. The organic phase was subjected to reduced pressure distillation (-0.1 MPa, 180°C) to remove light components, yielding 34 g of the compound of Formula XV (yield: about 32%), with a GC-detected purity of >93.4%.

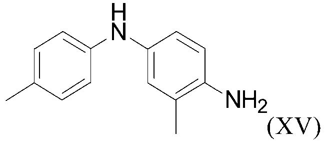

LC-MS(m/z): 212.30(M-H⁺).

### (2) Synthesis of Compound of Formula XIV

30 g (0.14 mol) of the compound of Formula XV, 104.5 g (1.8 mol) of acetone, and 0.8 g of Pt/C catalyst were charged into a reactor. The temperature was raised to 70°C, the atmosphere was replaced with hydrogen, and the pressure was increased to 1.5 MPa for reaction. The reaction was monitored by GC, and when the content of the compound of Formula XV was < 0.1%, the temperature was cooled down and the reaction was stopped. After filtration, the light components were removed by reduced pressure distillation (-0.1 MPa, 160°C) to obtain 34.7 g of the compound of Formula XIV (yield: about 97.5%), with a GC-detected purity of >95.8%. Property: dark red solid.

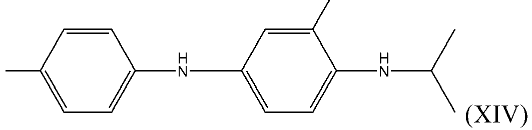

LC-MS(m/z): 240.35(M-H⁺).

### Examples 1-17 and Comparative Example 1

According to the recipes shown in Tables 1, 2, and 3, the rubber compositions of Examples 1-17 and Comparative Example 1 were prepared by the following process:
(1) add natural rubber and butadiene rubber into an internal mixer, then add carbon black, zinc oxide, stearic acid, aromatic oil, and an antidegradant (one or two selected from antidegradant 6PPD, Compound of Formula I, Compound of Formula II, Compound of Formula IV, Compound of Formula VI, Compound of Formula VIII, Compound of Formula X, Compound of Formula XII, and Compound of Formula XIV). Knead until the temperature of the rubber compound rises to 130°C, then discharge the rubber to obtain the first-stage rubber;
(2) after the first-stage rubber is cooled, mill it on an open mill, add accelerator NS and sulfur, and knead until the temperature of the rubber compound reaches 70°C, then sheet the rubber to obtain the second-stage rubber;
(3) vulcanize the second-stage rubber (145°C × 30 min) to obtain the vulcanized rubber.

**Table 1: Recipes of Rubber Compositions of Comparative Example 1 and Examples 1-5 (Unit: parts by mass)**

| Materials | Comparativ e Example 1 | Example 1 | Example 2 | Example 3 | Example 4 | Example 5 |
|---|---|---|---|---|---|---|
| SCR5 | 50 | 50 | 50 | 50 | 50 | 50 |
| BR9000 | 50 | 50 | 50 | 50 | 50 | 50 |
| Black carbon N550 | 50 | 50 | 50 | 50 | 50 | 50 |
| Zinc oxide | 5 | 5 | 5 | 5 | 5 | 5 |
| Stearic acid | 2 | 2 | 2 | 2 | 2 | 2 |
| Aromatic oil | 7 | 7 | 7 | 7 | 7 | 7 |
| Antidegradan t 6PPD | 2.0 | 0 | 0 | 0 | 0 | 0 |
| Compound of Formula I | 0 | 1.5 | 2.0 | 0 | 0 | 1.0 |
| Compound of Formula II | 0 | 0 | 0 | 1.5 | 2.0 | 1.0 |
| Sulfur | 1.5 | 1.5 | 1.5 | 1.5 | 1.5 | 1.5 |
| Accelerator NS | 0.8 | 0.8 | 0.8 | 0.8 | 0.8 | 0.8 |
| Total | 168.3 | 167.8 | 168.3 | 167.8 | 168.3 | 168.3 |

**Table 2: Recipes of Rubber Compositions of Examples 6-11 (Unit: parts by mass)**

| Materials | Example 6 | Example 7 | Example 8 | Example 9 | Example 10 | Example 11 |
|---|---|---|---|---|---|---|
| SCR5 | 50 | 50 | 50 | 50 | 50 | 50 |
| BR9000 | 50 | 50 | 50 | 50 | 50 | 50 |
| Black carbon N550 | 50 | 50 | 50 | 50 | 50 | 50 |
| Zinc oxide | 5 | 5 | 5 | 5 | 5 | 5 |
| Stearic acid | 2 | 2 | 2 | 2 | 2 | 2 |
| Aromatic oil | 7 | 7 | 7 | 7 | 7 | 7 |
| Compound of Formula I | 0.75 | 0.8 | 1.2 | 0 | 0 | 0 |
| Compound of Formula II | 0.75 | 1.2 | 0.8 | 0 | 0 | 0 |
| Compound of Formula IV | 0 | 0 | 0 | 1.0 | 0 | 0 |
| Compound of Formula VI | 0 | 0 | 0 | 1.0 | 0 | 0 |
| Compound of Formula VIII | 0 | 0 | 0 | 0 | 1.0 | 0 |
| Compound of Formula X | 0 | 0 | 0 | 0 | 1.0 | 0 |
| Compound of Formula XII | 0 | 0 | 0 | 0 | 0 | 1.0 |
| Compound of Formula XIV | 0 | 0 | 0 | 0 | 0 | 1.0 |
| Sulfur | 1.5 | 1.5 | 1.5 | 1.5 | 1.5 | 1.5 |
| Accelerator NS | 0.8 | 0.8 | 0.8 | 0.8 | 0.8 | 0.8 |
| Total | 167.8 | 168.3 | 168.3 | 168.3 | 168.3 | 168.3 |

**Table 3: Recipes of Rubber Compositions of Examples 12-17 (Unit: parts by mass)**

| Materials | Example 12 | Example 13 | Example 14 | Example 15 | Example 16 | Example 17 |
|---|---|---|---|---|---|---|
| SCR5 | 50 | 50 | 50 | 50 | 50 | 50 |
| BR9000 | 50 | 50 | 50 | 50 | 50 | 50 |
| Black carbon N550 | 50 | 50 | 50 | 50 | 50 | 50 |
| Zinc oxide | 5 | 5 | 5 | 5 | 5 | 5 |
| Stearic acid | 2 | 2 | 2 | 2 | 2 | 2 |
| Aromatic oil | 7 | 7 | 7 | 7 | 7 | 7 |
| Compound of Formula IV | 2 | 0 | 0 | 0 | 0 | 0 |
| Compound of Formula VI | 0 | 2 | 0 | 0 | 0 | 0 |
| Compound of Formula VIII | 0 | 0 | 2 | 0 | 0 | 0 |
| Compound of Formula X | 0 | 0 | 0 | 2 | 0 | 0 |
| Compound of Formula XII | 0 | 0 | 0 | 0 | 2 | 0 |
| Compound of Formula XIV | 0 | 0 | 0 | 0 | 0 | 2 |
| Sulfur | 1.5 | 1.5 | 1.5 | 1.5 | 1.5 | 1.5 |
| Accelerator NS | 0.8 | 0.8 | 0.8 | 0.8 | 0.8 | 0.8 |
| Total | 168.3 | 168.3 | 168.3 | 168.3 | 168.3 | 168.3 |

### Test Example 1: Flex Crack Resistance Test

The flex crack resistance test of the vulcanized rubber was carried out in accordance with the standard GB/T 13934-2006 "Vulcanized rubber or thermoplastic rubber - Determination of flex cracking and crack growth (De Mattia type)". The flex cycles corresponding to the early appearance of cracks (Grade 1, Grade 2) and sample damage (Grade 6) were recorded respectively. The results are shown in Tables 4, 5, and 6.

**Table 4: Flex Crack Resistance Test Results of Rubber Compositions of Comparative Example 1 and Examples 1-5**

| Crack Grade/ Flex Cycles (10,000 cycles) | Comparativ e Example 1 | Example 1 | Example 2 | Example 3 | Example 4 | Example 5 |
|---|---|---|---|---|---|---|
| 1 | 23.3 | 30.0 | 33.3 | 26.7 | 33.3 | 36.7 |
| | | +29% | +43% | +15% | +43% | +58% |
| 2 | 31.7 | 41.7 | 46.7 | 41.7 | 46.7 | 50.0 |
| | | +32% | +47% | +32% | +47% | +58% |
| 6 | 46.7 | 63.3 | 70.0 | 66.7 | 70.0 | 73.3 |
| | | +36% | +50% | +43% | +50% | +57% |

**Table 5: Flex Crack Resistance Test Results of Rubber Compositions of Examples 6-11**

| Crack Grade/ Flex Cycles (10,000 cycles) | Example 6 | Example 7 | Example 8 | Example 9 | Example 10 | Example 11 |
|---|---|---|---|---|---|---|
| 1 | 33.0 | 35.7 | 35.7 | 35.0 | 34.7 | 34.0 |
| | +42% | +53% | +53% | +50% | +49% | +46% |
| 2 | 42.7 | 49.7 | 50.0 | 46.0 | 48.3 | 46.7 |
| | +35% | +57% | +58% | 45% | 52% | +47% |
| 6 | 68.7 | 73.3 | 72.3 | 67.7 | 67.7 | 66.7 |
| | +47% | +57% | +55% | +45% | +45% | +43% |

**Table 6: Flex Crack Resistance Test Results of Rubber Compositions of Examples 12-17**

| Crack Grade/ Flex Cycles (10,000 cycles) | Example 12 | Example 13 | Example 14 | Example 15 | Example 16 | Example 17 |
|---|---|---|---|---|---|---|
| 1 | 32.0 | 31.7 | 31.7 | 30.7 | 31.3 | 30.3 |
| | +37% | +36% | +36% | +32% | +34% | +30% |
| 2 | 43.3 | 42.7 | 43.0 | 43.3 | 44.0 | 43.3 |
| | +37% | +35% | +36% | +37% | +39% | +37% |
| 6 | 62.7 | 62.3 | 63.3 | 62.7 | 63.0 | 62.0 |
| | +34% | +33% | +36% | +34% | +35% | +33% |

| | | | | | | |
|---|---|---|---|---|---|---|
| Note: The percentage values in Tables 4, 5, and 6 refer to the growth rate of the flex cycles of the corresponding Examples relative to that of Comparative Example 1. | | | | | | |

It can be seen from Tables 4-6 that the rubber compositions of Examples 1-17 (containing compounds of Formula A) have obvious advantages over the rubber composition of Comparative Example 1 (containing antidegradant 6PPD) in both early crack resistance and later crack growth rate reduction, with an average performance improvement of more than 30%. In addition, the flex crack resistance of Example 6 is significantly better than that of Examples 1 and 3; the flex crack resistance of Examples 5, 7, and 8 is significantly better than that of Examples 2 and 4; the flex crack resistance of Example 9 is significantly better than that of Examples 12 and 13; the flex crack resistance of Example 10 is significantly better than that of Examples 14 and 15; the flex crack resistance of Example 11 is significantly better than that of Examples 16 and 17. This indicates that the combination of two compounds of Formula A (e.g., combination of Compound of Formula I and Compound of Formula II, combination of Compound of Formula IV and Compound of Formula VI, combination of Compound of Formula VIII and Compound of Formula X, and combination of Compound of Formula XII and Compound of Formula XIV) exhibits a significant synergistic effect in improving flex crack resistance. Among them, the combination of Compound of Formula I and Compound of Formula II achieves the most excellent effect in improving flex crack resistance.

### Test Example 2: Dynamic Tensile Fatigue Resistance Test

The dynamic tensile fatigue resistance test of the vulcanized rubber was carried out in accordance with the standard GB/T 1688-2008 "Vulcanized rubber - Determination of tensile fatigue". The number of cycles of reciprocating motion of the sample under the test conditions of 50% tensile deformation and 5 Hz frequency until the sample fractures was recorded. The results are shown in Tables 7 and 8.

**Table 7: Dynamic Tensile Fatigue Resistance Test Results of Rubber Compositions of Comparative Example 1 and Examples 1-8**

| | Compar ative Exampl e 1 | Exam ple 1 | Exam ple 2 | Exam ple 3 | Exam ple 4 | Exam ple 5 | Exam ple 6 | Exam ple 7 | Exam ple 8 |
|---|---|---|---|---|---|---|---|---|---|
| Tensile Cycles at Fatigue Fracture (10,000 cycles) | 533 | 700 | 723 | 711 | 745 | 765 | 718 | 755 | 760 |
| | | +31% | +36% | +33% | +40% | +44% | +35% | +42% | +43% |

**Table 8: Dynamic Tensile Fatigue Resistance Test Results of Rubber Compositions of Examples 9-17**

| | Exam ple 9 | Exam ple 10 | Exam ple 11 | Exam ple 12 | Exam ple 13 | Exam ple 14 | Exam ple 15 | Exam ple 16 | Exam ple 17 |
|---|---|---|---|---|---|---|---|---|---|
| Tensile Cycles at Fatigue Fracture (10,000 cycles) | 723 | 728 | 718 | 698 | 689 | 695 | 679 | 700 | 683 |
| | +36% | +37% | +35% | +31% | +29% | +30% | +27% | +31% | +28% |

| | | | | | | | | | |
|---|---|---|---|---|---|---|---|---|---|
| Note: The percentage values in Tables 7 and 8 refer to the growth rate of the tensile cycles at fatigue fracture of the corresponding Examples relative to that of Comparative Example 1. | | | | | | | | | |

It can be seen from Tables 7 and 8 that the rubber compositions of Examples 1-17 (containing compounds of Formula A) have obvious advantages in dynamic tensile fatigue resistance over the rubber composition of Comparative Example 1 (containing antidegradant 6PPD), with an average performance improvement of more than 30%. In addition, the dynamic tensile fatigue resistance of Example 6 is better than that of Examples 1 and 3; the dynamic tensile fatigue resistance of Examples 5, 7, and 8 is better than that of Examples 2 and 4; the dynamic tensile fatigue resistance of Example 9 is better than that of Examples 12 and 13; the dynamic tensile fatigue resistance of Example 10 is better than that of Examples 14 and 15; the dynamic tensile fatigue resistance of Example 11 is better than that of Examples 16 and 17. This indicates that the combination of two compounds of Formula A (e.g., combination of Compound of Formula I and Compound of Formula II, combination of Compound of Formula IV and Compound of Formula VI, combination of Compound of Formula VIII and Compound of Formula X, and combination of Compound of Formula XII and Compound of Formula XIV) exhibits a synergistic effect in improving dynamic tensile fatigue resistance. Among them, the combination of Compound of Formula I and Compound of Formula II achieves the most excellent effect in improving dynamic tensile fatigue resistance.

## Claims

1. A rubber composition, wherein raw materials of the rubber composition comprise 100 parts by mass of a diene elastomer and 1-3 parts by mass of two or more compounds of Formula A: 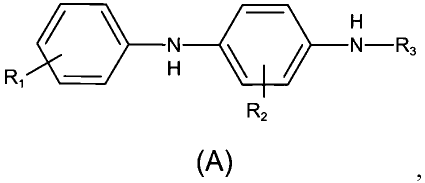 wherein, R₁, R₂, and R₃ in Formula A are each independently selected from C1-C8 alkyl.

2. The rubber composition according to claim 1,
wherein R₁ and R₂ in Formula A are each independently selected from C1-C3 alkyl, preferably each independently selected from methyl and ethyl; and/or
wherein R₃ in Formula A is selected from C3-C8 alkyl, preferably selected from C3-C6 alkyl, for example, selected from C4-C6 alkyl.

3. The rubber composition according to claim 1, wherein the raw materials of the rubber composition comprise two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV: 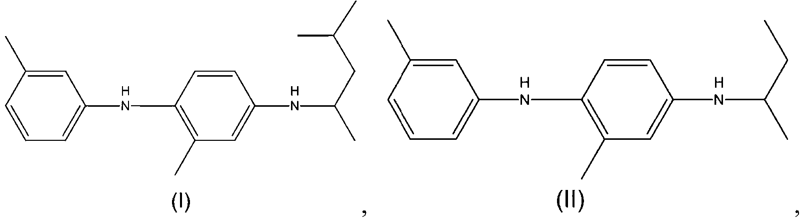 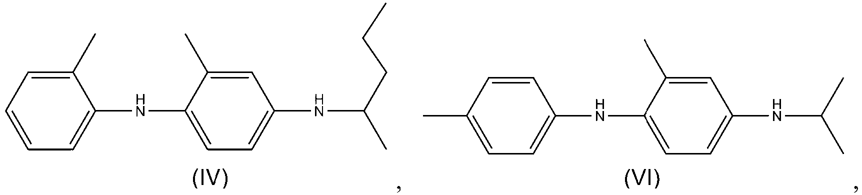 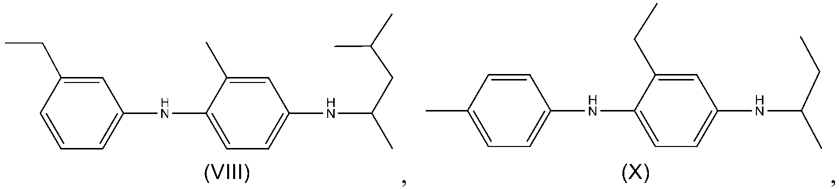 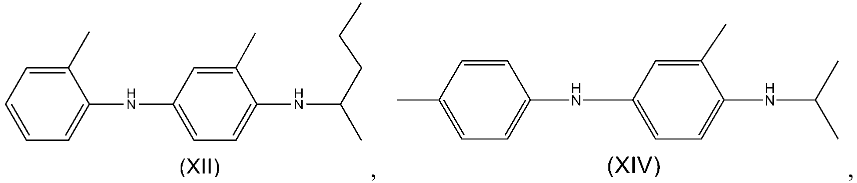 preferably, the raw materials of the rubber composition comprise Compound of Formula I and Compound of Formula II, or Compound of Formula IV and Compound of Formula VI, or Compound of Formula VIII and Compound of Formula X, or Compound of Formula XII and Compound of Formula XIV.

4. The rubber composition according to claim 3,
wherein in the raw materials of the rubber composition, a total dosage of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is 1-3 parts by mass, for example, 1.5-2 parts by mass; and/or
wherein in the raw materials of the rubber composition, a mass ratio of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is from 1:2 to 2:1, for example, from 1:1.5 to 1.5:1.

5. The rubber composition according to claim 1, wherein it has one or more of the following characteristics:
wherein the diene elastomer comprises natural rubber and butadiene rubber in a mass ratio of from 1:2 to 2:1;
wherein the raw materials of the rubber composition further comprise 30-70 parts by mass of a reinforcing filler; the reinforcing filler is preferably carbon black;
wherein the raw materials of the rubber composition further comprise 0.5-3 parts by mass of sulfur;
wherein the raw materials of the rubber composition further comprise 1-10 parts by mass of an activator; preferably, the activator is zinc oxide;
wherein the raw materials of the rubber composition further comprise 2-15 parts by mass of a softener;
wherein the raw materials of the rubber composition further comprise 0.2-2 parts by mass of an accelerator; the accelerator is preferably N-tert-butyl-2-benzothiazolesulfenamide.

6. A rubber article, wherein the rubber article comprises the rubber composition according to any one of claims 1-4; preferably, the rubber article is a tire.

7. A method for improving the fatigue aging resistance of a rubber composition, wherein the method comprises: based on 100 parts by mass of the diene elastomer contained in the raw materials of the rubber composition, adding 1-3 parts by mass of two or more compounds of Formula A to the raw materials of the rubber composition: 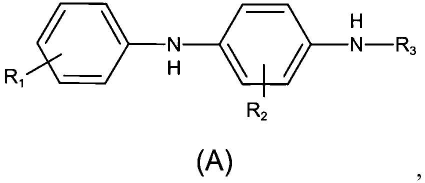
wherein R₁, R₂, and R₃ in Formula A are each independently selected from C1-C8 alkyl;
preferably, wherein R₁ and R₂ in Formula A are each independently selected from C1-C3 alkyl, preferably each independently selected from methyl and ethyl;
preferably, wherein R₃ in Formula A is selected from C3-C8 alkyl, preferably selected from C3-C6 alkyl, for example, selected from C4-C6 alkyl.

8. The method according to claim 7, wherein the method comprises: adding two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV to the raw materials of the rubber composition: 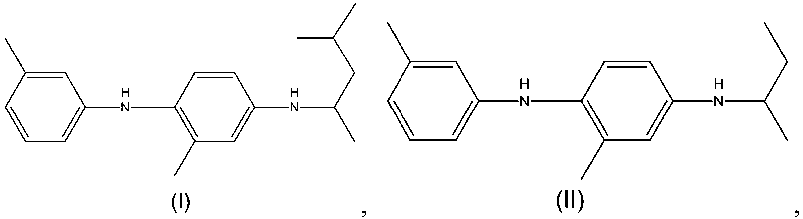 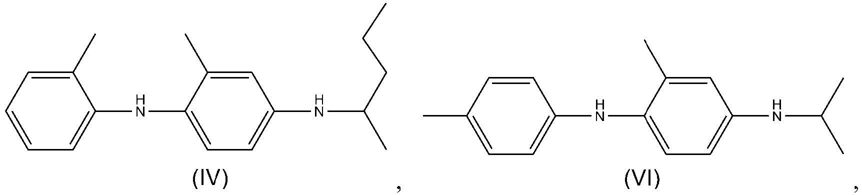 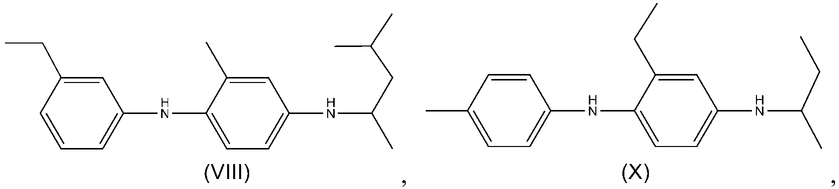 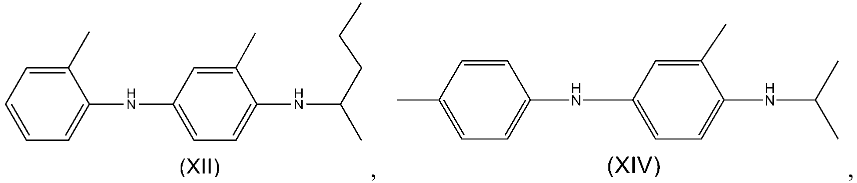 preferably, the method comprises: adding Compound of Formula I and Compound of Formula II, or Compound of Formula IV and Compound of Formula VI, or Compound of Formula VIII and Compound of Formula X, or Compound of Formula XII and Compound of Formula XIV to the raw materials of the rubber composition.

9. The method according to claim 8,
wherein in the raw materials of the rubber composition, a total dosage of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is 1-3 parts by mass, for example, 1.5-2 parts by mass; and/or
wherein in the raw materials of the rubber composition, a mass ratio of the two compounds selected from the group consisting of Compound of Formula I, Compound of Formula II, Compound of Formula IV, Compound of Formula VI, Compound of Formula VIII, Compound of Formula X, Compound of Formula XII, and Compound of Formula XIV is from 1:2 to 2:1, for example, from 1:1.5 to 1.5:1.

10. The method according to claim 7, wherein the method comprises one or more of the following characteristics:
wherein the diene elastomer comprises natural rubber and butadiene rubber in a mass ratio of from 1:2 to 2:1;
wherein the raw materials of the rubber composition further comprise 30-70 parts by mass of a reinforcing filler; the reinforcing filler is preferably carbon black;
wherein the raw materials of the rubber composition further comprise 0.5-3 parts by mass of sulfur;
wherein the raw materials of the rubber composition further comprise 1-10 parts by mass of an activator; preferably, the activator is zinc oxide;
wherein the raw materials of the rubber composition further comprise 2-15 parts by mass of a softener;
wherein the raw materials of the rubber composition further comprise 0.2-2 parts by mass of an accelerator; the accelerator is preferably N-tert-butyl-2-benzothiazolesulfenamide.
